# EUROPÄISCHE PATENTSCHRIFT

(11) **EP 1 355 579 B1**
(45) Veröffentlichungstag und Bekanntmachung des Hinweises auf die Patenterteilung: **16.03.2011**
(21) Anmeldenummer: 02702279.7
(22) Anmeldetag: 17.01.2002
(51) Int. Cl.: A61B 18/12

(54) **VORRICHTUNG ZUR ELEKTROTHERMISCHEN BEHANDLUNG DES MENSCHLICHEN ODER TIERISCHEN KÖRPERS**
DEVICE FOR THE ELECTROTHERMAL TREATMENT OF THE HUMAN OR ANIMAL BODY
DISPOSITIF DE TRAITEMENT ELECTROTHERMIQUE DU CORPS HUMAIN OU ANIMAL

(30) Priorität: 19.01.2001 DE 10102254
(43) Veröffentlichungstag der Anmeldung: 29.10.2003
(73) Patentinhaber: Celon AG Medical Instruments, 14513 Teltow (DE)
(72) Erfinder: DESINGER, Kai, 12157 Berlin (DE); FRICKE, Thomas, 10245 Berlin (DE); STEIN, Thomas, 10787 Berlin (DE)
(74) Vertreter: Eisenführ, Speiser & Partner
(86) Internationale Anmeldenummer: PCT/EP2002/000459
(87) Internationale Veröffentlichungsnummer: WO 2002/056782

(56) Entgegenhaltungen:
- EP-A- 0 870 473
- WO-A-95/09577
- DE-A- 3 623 340
- DE-A- 3 904 558
- US-A- 5 370 645
- US-A- 5 423 808
- US-A- 5 514 129

## Beschreibung

Die Erfindung betrifft eine Vorrichtung zur elektrothermischen Behandlung des menschlichen oder tierischen Körpers, insbesondere zur Elektrokoagulation oder Elektrotomie, gemäß dem Oberbegriff des Anspruches 1.

Beispielsweise aus der WO 97/17009 ist eine derartige Vorrichtung bekannt, bei der zur Gewebekoagulation und zur Gewebetrennung hochfrequente Wechselströme im Frequenzbereich zwischen etwa 300 kHz bis einigen MHz verwendet werden, wodurch das behandelte Gewebe koaguliert bzw. vaporisiert wird, was als Elektrokoagulation bzw. Elektrotomie bezeichnet wird. Zum Einsatz gelangt dabei eine Sondenanordnung, bei der auf einem länglichen, stabförmigen Träger mindestens zwei Elektroden voneinander beabstandet und isoliert angeordnet sind und mit einem extrakorporal angeordneten Hochfrequenz-Generator mit der notwendigen HF-Leistung versorgt werden, so dass sich zwischen den Elektroden ein ausreichendes elektrisches oder elektromagnetisches Feld ausbildet, welches auf die unmittelbare Umgebung der Elektroden begrenzt ist und das Körpergewebe koaguliert oder vaporisiert, welches zwischen den beiden Elektroden im Wirkbereich des elektromagnetischen und des daraus resultierenden thermischen Feldes liegt.

Es hat sich gezeigt, dass die Änderung der zwischen den Elektroden messbaren elektrischen HF-Impedanz während des Behandlungsvorganges, d.h. während des Koagulationsprozesses des Gewebes nach einem im wesentlichen gleichbleibenden Schema abläuft. Während der Absolutwert der zu messenden HF-Impedanz zwischen den Elektroden von verschiedenen Einflussgrößen, wie z.B. der Applikatorgeometrie, der Gewebebeschaffenheit, abhängt, so besitzt diese HF-Impedanz einen typischen zeitlichen Verlauf, der dadurch gekennzeichnet ist, dass er nach einer bestimmten Behandlungszeit stark ansteigt, wobei das Körpergewebe im Bereich des betreffenden elektromagnetischen Feldes koaguliert und dadurch austrocknet, wodurch die Impedanz der einzelnen Gewebezellen wesentlich erhöht wird.

Die Zusammenhänge zwischen dem zeitlichen Verlauf der HF-Impedanz und dem Koagulationsprozess sind schon seit längerer Zeit bekannt und haben unter anderem zur Konstruktion von Regelkreisen für Hochfrequenzgeneratoren geführt, welche dazu ausgebildet sind, über die Messung der HF-Impedanz des Körpergewebes das Fortschreiten des Koagulationsprozesses zu kontrollieren. So wird zum Beispiel in der WO 95/09577 ein Regelkreis für einen Hochfrequenzgenerator offenbart, der dazu ausgebildet ist, eine benutzerdefinierte Leistungsabgabe des Generators mit Hilfe des zeitlichen HF-Impedanzverlaufes während der Behandlung zu kontrollieren.

Während des Koagulationsprozesses kommt es zunächst zur Austrocknung des Gewebes. Diese Austrocknung der Gewebezellen schreitet rapide fort, bis schließlich die Elektroden von ausgetrocknetem Gewebe umgeben sind. Die Austrocknung des Gewebes und die dadurch bedingte erhebliche Vergrößerung der elektrischen HF-Impedanz verursacht ihrerseits einen Einbruch der Generatorleistung durch Fehlanpassung. Beim Abschalten des Generators sinkt dann die Impedanz exponentiell nahezu auf ihren Minimalwert, weil der Leistungseintrag in das Gewebe gestoppt wird und kein Verdampfen von Gewebeflüssigkeit mehr stattfindet. Dies hat zur Folge, dass sich die dehydrierten Bereiche wieder mit Gewebewasser füllen, dadurch die Impedanz entsprechend sinkt. Wird anschließend die Generatorleistung wieder aktiviert, kommt es nach einem bestimmten Zeitintervall zu einem erneuten Impedanzanstieg, bis der Zustand vor dem Ausschalten des Generators erreicht war. Dieser beobachtete Verlauf der elektrischen Impedanz zwischen den beiden Elektroden bzw. der dadurch bedingte zeitliche Verlauf der abgegebenen Leistung des HF-Generators kann als Information nützlich sein, damit der Benutzer den Behandlungsvorgang besser gestalten kann.

Aufgabe der Erfindung ist es daher, eine Vorrichtung der eingangs genannten Art derart weiterzubilden, dass eine verbesserte Behandlung, und insbesondere eine genauere Bestimmung der Behandlungsdauer, möglich ist.

Diese Aufgabe wird erfindungsgemäß bei der Vorrichtung der eingangs genannten Art gelöst durch eine Signalquelle, die ein Signal abgibt, welches während eines Behandlungsvorganges dem Benutzer Information über den Zustand des zwischen den beiden Elektroden befindlichen Körpergewebes übermittelt.

Die Vorteile der Erfindung liegen insbesondere darin, dass ein Signal abgeleitet und für den Benutzer wahrnehmbar gemacht wird,, welches den Benutzer während des Behandlungsvorganges darüber informiert, in welchem Zustand sich das Körpergewebe zwischen den beiden Elektroden befindet.

Besonders bevorzugt greift die Signalquelle von dem HF-Generator eine Größe ab, die dem abgegebenen HF-Strom proportional ist, und da außerdem eine weitere Größe ab, die zur abgegebenen HF-Spannung proportional ist. Aus diesen beiden abgegriffenen Größen wird erfindungsgemäß die HF-Leistung des Generators und/oder die HF-Impedanz zwischen den Elektroden der Applikationsanordnung berechnet, und das von der Signalquelle abgegebene Signal hängt von der berechneten HF-Leistung oder der berechneten elektrischen Impedanz ab, so dass der Benutzer über die HF-Impedanz, die zwischen den beiden Elektroden gemessen wird, und die ein integrales Maß über das Fortschreiten der Gewebekoagulation ist, während eines Behandlungsvorganges ständig auf dem laufenden gehalten wird.

Besonders bevorzugt gibt die Signalquelle ein Kennsignal, wenn die elektrische HF-Impedanz zwischen den beiden Elektroden einen vorgegebenen Wert überschreitet, bei dem die Gewebekoagulation im Bereich der Elektroden zum Abschluss gekommen ist, so dass dann der Benutzer den Applikator entweder in eine andere örtliche Position innerhalb des Körpergewebes verbringt oder den Vorgang beenden kann.

Besonders bevorzugt ist die Signalquelle eine akustische Signalquelle, die ein hörbares Signal abgibt. Die Frequenz des abgegebenen Signales hängt bevorzugt vom zeitlichen Verlauf der abgegebenen HF-Leistung des HF-Generators ab oder alternativ von dem zeitlichen Verlauf der HF-Impedanz, die zwischen den beiden Elektroden vorliegt. Bei dieser Ausführungsform der Erfindung kann der Benutzer anhand der Tonhöhe die Veränderung des Gewebes erkennen. Nimmt die Frequenz des hörbaren Signals zunehmender im HF-Impedanz des Körpergewebes ebenfalls zu, und das Signal geht in das Kennsignal über, wenn die Impedanz des Körpergewebes zwischen den beiden Elektroden einen vorgegebenen Wert übersteigt bzw. wenn die Leistungsabgabe des HF-Generators unter einen vorgegebenen Wert fällt. Das Kennsignal kann ein akustisches Signal mit konstanter Frequenz sein, alternativ kann es auch als zeitlich moduliertes, beispielsweise als impulsförmiges Hörsignal etc. ausgebildet sein, welches dem Benutzer auffällig das Überschreiten der vorgegebenen Impedanzgrenze angibt. Gemäß einer weiteren bevorzugten Ausführungsform der Erfindung gibt die Signalquelle ein Abschaltsignal ab, welches den HF-Generator abschaltet oder von den Elektroden trennt, wenn die elektrische Impedanz zwischen Elektroden den vorgegebenen Schwellwert überschreitet bzw. die entsprechende abgegebene HF-Leistung des Generators den vorgegebenen Wert unterschreitet.

Vorteilhafte Weiterbildungen der Erfindung sind durch die Merkmale der Unteransprüche gekennzeichnet.

Im folgenden wird eine Ausführungsform der Erfindung anhand der Zeichnungen näher erläutert. Es zeigen:
- Figur 1: eine schematische Darstellung der Vorrichtung;
- Figur 2: ein schematisches Schaltbild der Vorrichtung;
- Figur 3: eine schematische Darstellung eines Koagulationsprozesses während eines Behandlungsvorganges; und
- Figur 4: einen schematischen Verlauf der zwischen den Elektroden des Appli- kators vorliegenden HF-Impedanz des Körpergewebes während eines Behandlungsvorganges.

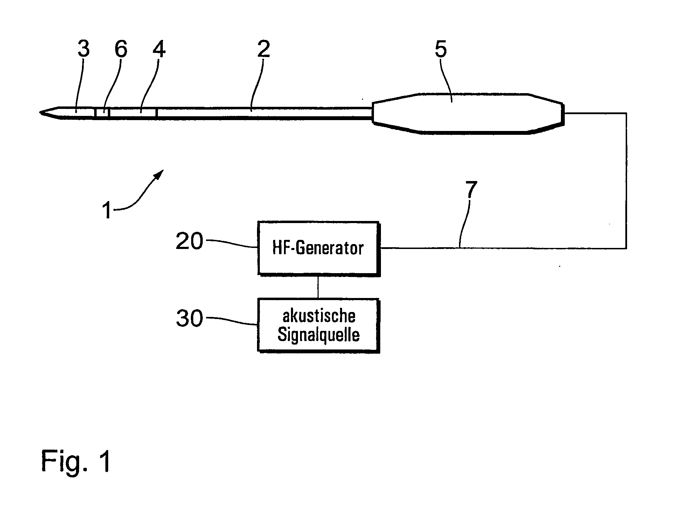

Figur 1 zeigt eine Vorrichtung zur elektro-thermischen Behandlung des menschlichen oder tierischen Körpers, die einen Applikator 1 enthält, der einen elektrisch isolierten Schaft 2 sowie ein Elektrodenteil, bestehend aus einer am freien Ende spitz zulaufenden distalen Elektrode 3 und einer proximalen Elektrode 4, besitzt. Die Elektroden 3 und 4 bilden einen Bestandteil des Applikators 1 und sind gegeneinander durch ein isolierendes Abstandselement 6 voneinander getrennt. An das proximale Ende des Schaftes schließt sich das Handstück mit der elektrischen Zuleitung 7 an. Die Elektroden 3 und 4 sind über die Zuleitung 7 mit dem HF-Generator 20 verbunden. Der Applikator 1 lässt sich an einem Griffteil 5 von dem Benutzer halten und während eines Behandlungsvorganges führen.

Der HF-Generator 20 ist mit einer akustischen Signalquelle 30 versehen, die während eines Brandungsvorganges von dem HF-Generator die elektrischen Ausgangsgrößen abgreift oder abtastet und ein Signal erzeugt, welches dem Benutzer Informationen über den Zustand des zwischen den beiden Elektroden im Therapiegebiet befindlichen Körpergewebes übermittelt.

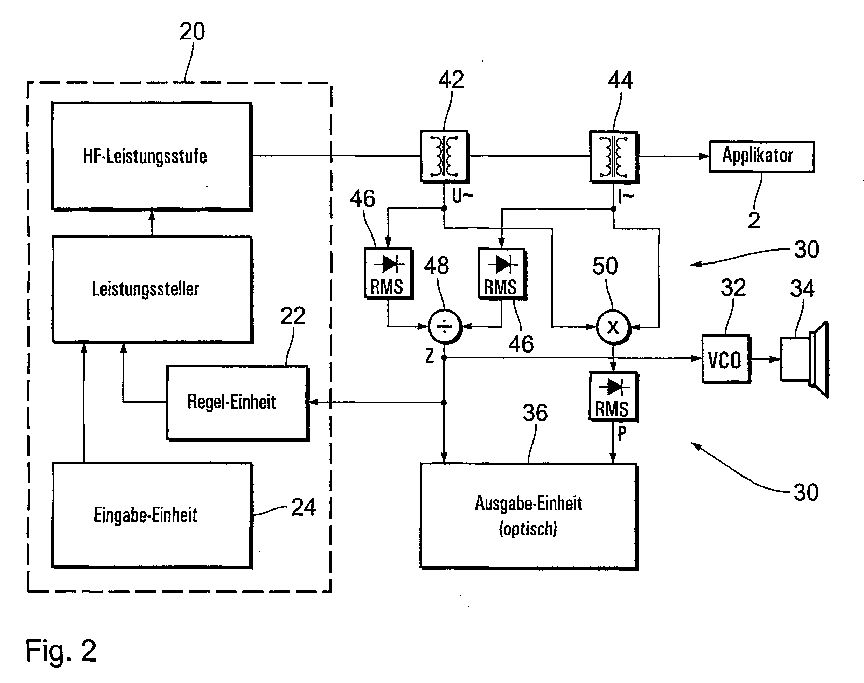

Wie insbesondere Figur 2 entnehmbar ist, wird über das Koppelglied 42 und das Koppelglied 44 Spannung und Strom von dem HF-Generator 20 zum Applikator 2 geführten HF-Leistung abgegriffen, und aus diesen Messgrößen wird über Gleichrichtungsstufen 46 mittels eines Dividiergliedes 48 die elektrische Impedanz ermittelt, die sich aus der zwischen den beiden Elektroden 3, 4 anliegenden Spannung und dem zwischen den Elektroden 3, 4 fließenden Strom ergibt, die also Informationen über den Zustand des zwischen den beiden Elektroden befindlichen Körpergewebes darstellt. Zusätzlich wird über ein Multiplizierglied 50 die HF-Leistung gewonnen, die über den Applikator in das Therapiegebiet eingeleitet wird.

Ein von der berechneten elektrischen Impedanz z abgeleitetes, beispielsweise proportionales Signal wird dann über eine elektrische Schaltung 32 einem Lautsprecher 34 zugeführt, der ein akustisches, hörbares Signal abgibt, dessen Frequenz von der elektrischen HF-Impedanz des Körpergewebes zwischen den Elektroden 3, 4 abhängt. Beispielsweise nimmt die Frequenz des vom Lautsprecher 34 abgegebenen Signals zu, wenn die Impedanz zunimmt. Überschreitet die Impedanz z einen vorgegebenen Schwellwert, so geht das akustische Signal in ein Tonsignal mit konstanter Frequenz über, welches als Kennsignal dem behandelnden Arzt anzeigt, dass das Körpergewebe zwischen den Elektroden 3, 4 koaguliert und in starkem Maße dehydriert ist, so dass die Behandlung an dem betreffenden Behandlungsort endet, der Applikator 2 also entweder zu einem anderen Behandlungsort verschoben oder aus dem Körpergewebe zurückgezogen werden kann.

Die Abtasteinrichtungen 42, 44, die Gleichrichterschaltungen 46, das Dividierglied 48 und gegebenenfalls das Multiplizierglied 50 stellen zusammen mit der elektrischen Schaltung 32 und dem Lautsprecher 34 die Signalquelle 30 dar, der in der dargestellten Ausführungsform noch eine optische Ausgabeeinheit 36 hinzugefügt ist, welche die elektrische HF-Impedanz zwischen den Elektroden 2, 3 und/oder die an die Elektroden 2, 3 abgegebene Leistung P und/oder - statt des akustischen Hörsignals - ein optisches Informationssignal dem Benutzer anzeigen kann, welches von der Impedanz zwischen den Elektroden 3, 4 und/oder der an den Elektroden abgegebenen Leistung P abhängen kann.

Von dem Ausgangssignal der Signalquelle 30, welches von der abgegebenen HF-Leistung des Generators oder der elektrischen HF-Impedanz des Körpergewebes zwischen den Elektroden 3, 4 abhängt, wird gemäß Figur 2 ein Regelsignal abgeleitet, welches in den HF-Generator 20 zurückgeführt wird und dort in einer Regeleinheit 22 so aufbereitet wird, dass es dann zur Regelung der abgegebenen HF-Leistung dient. Beispielsweise kann durch die Regeleinheit 22 im Generator 20 ein Steuersignal erzeugt werden, sobald die Impedanz nach einem vorausgegangenen *Impedanzanstieg wieder unter einen vorgegebenen absoluten oder relativen Wert sinkt, welches den Generator 20 wieder aktiviert, um dadurch den Koagulationsprozeß fortzusetzen. Alternativ lässt sich bei der Regelung ein bestimmter Impedanzwert als Sollwert vorgeben, und die abgegebene Leistung des HF-Generators 20 lässt sich so einregeln, dass sich der tatsächliche Impedanzwert (Istwert) dem vorgegebenen Impedanzwert (Sollwert) in gewünschter Weise kontinuierlich oder in Intervallen annähert.

In einer bevorzugten Ausführungsform der Erfindung gestattet es die Eingabeeinheit 24 des HF-Generators 20, ein Leistungs-/Zeitprofil vorzugeben, dem die abgegebene Leistung des HF-Generators 20 unterliegt.

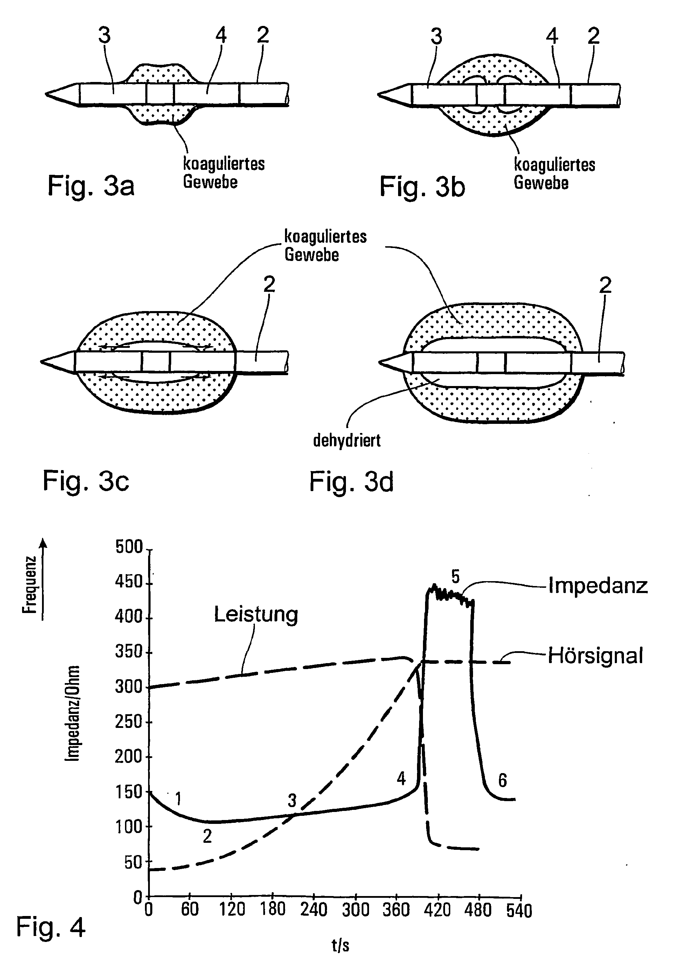

Figuren 3a bis 3d zeigen das Körpergewebe im Bereich der Elektroden 3, 4 des Applikators 2 zeitlich fortschreitender Behandlung und damit Vergrößerung der koagulierten Gewebeareale. Der Koagulierungsprozeß beginnt in dem angrenzenden Körpergewebe, welches im Bereich der einander benachbarten Zonen der Elektroden 3, 4 angesiedelt ist. Das Koagulierungsareal breitet sich dann nach vorn zur freien Spitze des Applikators 2 und nach proximal zum proximalen Ende der Elektrode 4 aus, vgl. auch die Pfeile in Figur 3c. Bei zunehmender Behandlungszeit entsteht in der unmittelbaren Nähe der Elektroden dann eine dehydrierte Zone, welche sich schließlich über die Länge beider Elektroden 3, 4 ausdehnt. Mit der Bildung der dehydrierten Gewebezone um die Elektroden 3, 4 herum geht eine erhebliche Erhöhung der elektrischen Impedanz z einher, die zwischen den Elektroden 3, 4 gemessen wird. Die Impedanz erreicht ihr Maximum bei Vorliegen einer Konfiguration gemäß Figur 3d, da dann nahezu die gesamte Zone zwischen den beiden Elektroden von einer dehydrierten Zone gebildet ist. Diese extreme Vergrößerung der Impedanz verursacht eine starke Verringerung der abgegebenen Generatorleistung aufgrund der Fehlanpassung, die durch die Impedanzerhöhung hervorgerufen ist. Der Verlauf der Impedanz gibt Auskunft über den Fortschritt des Koagulationsprozesses im umliegenden Gewebe. An dem Impedanzverlauf ist erkennbar, wann der Applikator 2 das für ihn - abhängig vom Applikatoraufbau und der HF-Leistung -maximal generierbare Koagulationsvolumen erreicht hat.

Wie insbesondere Figur 4 entnehmbar ist, steigt die Impedanz an Punkt 4 der Impedanzkurve stark an bis zu einem maximalen Wert an der Stelle 5. Das von der Signalquelle 30 erzeugte und über einen Lautsprecher 34 abgegebene Hörsignal - in der Darstellung gemäß Figur 4 - nimmt mit seiner Frequenz stetig zu und geht dann, wenn die Impedanz des Körpergewebes zwischen den Elektroden 3, 4 über einen vorgegebenen Schwellwert steigt, eine konstante Frequenz an, die dem behandelnden Arzt anzeigt, dass das maximal generierbare Koagulationsvolumen erreicht ist, der Generator entweder abgeschaltet oder der Applikator 1 in eine andere Behandlungsposition geschoben werden kann. Beim Abschalten des Generators 20 sinkt die Impedanz sehr rasch wieder auf ihren Minimalwert, weil der Leistungseintrag in das Gewebe gestoppt ist und kein Verdampfen von Gewebewasser mehr stattfindet, sondern sich die dehydrierten Gewebebereiche wieder mit Gewebewasser anfüllen, vgl. Punkt 6 des Impedanzverlaufes. Wird dann der Generator noch einmal zugeschaltet und der Koagulationsvorgang fortgesetzt, so steigt die Impedanz sehr rasch wieder auf ihren Maximalwert, der durch eine Dehydrierung der von dem elektromagnetischen Feld durchdrungenen Gewebebereich gekennzeichnet ist.

## Patentansprüche

1. Vorrichtung zur elektrothermischen Behandlung des menschlichen oder tierischen Körpers, insbesondere zur Gewebekoagulation oder Elektrotomie,
mit einem länglichen Applikator (1)
mit mindestens zwei Elektroden (3, 4) zur Einführung in den zu behandelnden Körper, wobei die beiden Elektroden (3, 4) zur Erzeugung eines das Körpergewebe im Behandlungsgebiet erwärmenden elektrischen oder elektromagnetischen Feldes auf dem Applikator elektrisch gegeneinander isoliert und zueinander beabstandet angeordnet sind und über jeweils eine Zuleitung mit einem extrakorporal angeordneten Hochfrequenz-Generator (20) verbunden sind,
und mit einer Signalquelle (30), die ein Signal abgibt, welches während eines Behandlungsvorganges dem Benutzer Information über den Zustand des zwischen den beiden Elektroden (3, 4) befindlichen Körpergewebes übermittelt,
**dadurch gekennzeichnet,**
**dass** die Frequenz des von der Signalquelle (3, 4) abgegebenen Signales zunimmt, wenn die Impedanz des zwischen den Elektroden (3, 4) liegenden Körpergewebes während eines Behandlungsvorganges zunimmt.

2. Vorrichtung nach Anspruch 1,
**dadurch gekennzeichnet, dass** die Frequenz des von der Signalquelle (30) abgegebenen Signals näherungsweise proportional zu der Impedanz des Körpergewebes zwischen den Elektroden (3, 4), bzw. umgekehrt proportional zu der abgegebenen HF-Leistung des HF-Generators (20) ist.

3. Vorrichtung zur elektrothermischen Behandlung des menschlichen oder tierischen Körpers, insbesondere zur Gewebekoagulation oder Elektrotomie,
mit einem länglichen Applikator (1)
mit mindestens zwei Elektroden (3, 4) zur Einführung in den zu behandelnden Körper, wobei die beiden Elektroden (3, 4) zur Erzeugung eines das Körpergewebe im Behandlungsgebiet erwärmenden elektrischen oder elektromagnetischen Feldes auf dem Applikator elektrisch gegeneinander isoliert und zueinander beabstandet angeordnet sind und über jeweils eine Zuleitung mit einem extrakorporal angeordneten Hochfrequenz-Generator (20) verbunden sind,
und mit einer Signalquelle (30), die ein Signal abgibt, welches während eines Behandlungsvorganges dem Benutzer Information über den Zustand des zwischen den beiden Elektroden (3, 4) befindlichen Körpergewebes übermittelt,
**dadurch gekennzeichnet,**
**dass** die Frequenz des von der Signalquelle (30) abgegebenen Signales abnimmt, wenn die Impedanz des Körpergewebes zwischen den beiden Elektroden (3, 4) zunimmt.

4. Vorrichtung nach einem der vorstehenden Ansprüche,
**dadurch gekennzeichnet, dass** die Signalquelle (30) eine akustische Signalquelle ist, die ein hörbares Signal abgibt, dessen Frequenz von dem zeitlichen Verlauf der abgegebenen HF-Leistung des HF-Generators (20) oder vom zeitlichen Verlauf der HF-Impedanz zwischen den Elektroden (3, 4) abhängt.

5. Vorrichtung nach einem der Ansprüche 1 bis 4,
**dadurch gekennzeichnet, dass** die Signalquelle ein Kennsignal abgibt, wenn die elektrische HF-Impedanz, die das Körpergewebe zwischen den beiden Elektroden (3, 4) während eines Behandlungsvorganges aufweist, einen vorgegebenen Wert überschreitet.

6. Vorrichtung nach einem der Ansprüche 1 bis 4,
**dadurch gekennzeichnet, dass** die Signalquelle (30) ein Kennsignal abgibt, wenn die abgegebene HF-Leistung des HF-Generators (20) während eines Behandlungsvorganges einen vorgegebenen Wert unterschreitet.

7. Vorrichtung nach einem der vorstehenden Ansprüche,
**dadurch gekennzeichnet, dass** sich das von der Signalquelle (30) abgegebene hörbare Signal plötzlich ändert, wenn die Impedanz des zwischen den Elektroden (3, 4) befindlichen Körpergewebes während eines Behandlungsvorganges über einen vorgegebenen Wert steigt, bzw. wenn die Leistungsabgabe des HF-Generators (20) unter einen vorgegebenen Wert fällt.

8. Vorrichtung nach einem der vorstehenden Ansprüche,
**dadurch gekennzeichnet, dass** das hörbare Signal, welches von der Signalquelle (30) abgegeben wird, getaktet wird, wenn die Impedanz des zwischen den Elektroden (3, 4) befindlichen Körpergewebes während eines Behandlungsvorganges über einen vorgegebenen Wert steigt, bzw. wenn die Leistungsabgabe des HF-Generators (20) unter einen vorgegebenen Wert fällt.

9. Vorrichtung nach einem der Ansprüche 1 bis 7,
**dadurch gekennzeichnet, dass** die Signalquelle (30) während eines Behandlungsvorganges von dem HF-Generator eine zum abgegebenen HF-Strom und zu der abgegebenen HF-Spannung proportionalen Größen abgreift, aus diesen Größen die abgegebene HF-Leistung des Generators (20) und/oder die HF-Impedanz zwischen den Elektroden (3, 4) berechnet und ein Signal abgibt, welches von der berechneten abgegebenen HF-Leistung des Generators (20) und/oder von der berechneten elektrischen HF-Impedanz abhängt, die das Körpergewebe zwischen den beiden Elektroden (3, 4) aufweist.

10. Vorrichtung nach Anspruch 1 oder 3,
**dadurch gekennzeichnet, dass** von dem Signal der Signalquelle (30), welches von der abgegebenen HF-Leistung des HF-Generators (20) oder der elektrischen HF-Impedanz zwischen den Elektroden (3, 4) abhängt, ein Signal zur Regelung einer der elektrischen Ausgangsgrößen des HF-Generators (20) abgeleitet und zum HF-Generator (20) zurückgeführt wird.

11. Vorrichtung nach Anspruch 10,
**dadurch gekennzeichnet, dass** die abgegebene Leistung des HF-Generators (20) derart geregelt wird, dass bei einem Ansteigen des Impedanz-Wertes ein Überschreiten eines vorgegebenen Wertes unterbleibt.

12. Vorrichtung nach einem der vorstehenden Ansprüche,
**dadurch gekennzeichnet, dass** die akustische Signalquelle (30) ein Abschaltsignal dem HF-Generator (20) zuführt, welches den HF-Generator (20) von den Elektroden (3, 4) trennt bzw. abschaltet, wenn die elektrische Impedanz des zwischen den Elektroden (3, 4) liegenden Körpergewebes während eines Behandlungsvorganges einen vorgegebenen absoluten oder relativen Wert überschreitet.

13. Vorrichtung nach einem der vorstehenden Ansprüche,
**dadurch gekennzeichnet, dass** die Signalquelle (30) dem HF-Generator (20) ein Signal zuführt, welches den HF-Generator (20) wieder aktiviert bzw. mit den Elektroden (3, 4) verbindet, wenn die Impedanz des zwischen den Elektroden 3, 4 liegenden Körpergewebes nach einem vorangegangenen Impedanzanstieg wieder einen vorgegebenen absoluten oder relativen Wert unterschreitet.

14. Vorrichtung nach einem der vorstehenden Ansprüche,
**dadurch gekennzeichnet, dass** die abgegebene Leistung des HF-Generators (20) nach einem vom Benutzer vorgebbaren Zeit-Leistungsprofil gesteuert wird.

15. Vorrichtung nach einem der vorstehenden Ansprüche,
**dadurch gekennzeichnet, dass** die akustische Signalquelle (30) in dem HF-Generator (20) enthalten ist.

16. Vorrichtung nach einem der vorstehenden Ansprüche,
**dadurch gekennzeichnet, dass** der längliche Applikator (2), der die Elektroden (3, 4) trägt, als Stab mit einem konstanten oder variablen Querschnitt ausgebildet ist und an seinem freien vorderen Ende spitz zuläuft.

## Claims

1. Device for electrothermal treatment of the human or animal body, in particular for tissue coagulation or electrotomy,
comprising an elongate applicator (1)
having at least two electrodes (3, 4) for insertion into the body to be treated, wherein to produce an electrical or electromagnetic field for heating the body tissue in the treatment region the two electrodes (3, 4) are arranged on the applicator electrically insulated from one another and spaced apart from one another and are each connected by way of a respective feed line to a highfrequency generator (20) arranged outside the body,
and a signal source (30) which emits a signal which during a treatment process provides the user with information about the condition of the body tissue between the two electrodes (3, 4),
**characterised in that**
the frequency of the signal emitted by the signal source (3,4) increases when the impedance of the body tissue between the electrodes (3, 4) increases during a treatment process.

2. Device according to claim 1,
**characterised in that** the frequency of the signal emitted by the signal source (30) is almost proportional to the impedance of the body tissue between the electrodes (3, 4) or conversely is proportional to the emitted HF-power of the HF-generator (20).

3. Device for the electrothermal treatment of the human or animal body, in particular for tissue coagulation or electrotomy,
comprising an elongate applicator (1)
having at least two electrodes (3, 4) for insertion into the body to be treated, wherein to produce an electrical or electromagnetic field for heating the body tissue in the treatment region the two electrodes (3, 4) are arranged on the applicator electrically insulated from one another and spaced apart from one another and are each connected by way of a respective feed line to a highfrequency generator (20) arranged outside the body,
and a signal source (30) which emits a signal which during a treatment process provides the user with information about the condition of the body tissue between the two electrodes (3, 4),
**characterised in that** the frequency of the signal emitted by the signal source (3,4) decreases when the impedance of the body tissue between the two electrodes (3, 4) increases.

4. Device according to one of the preceding claims,
**characterised in that** the signal source (30) is an acoustic signal source which emits an audible signal, whose frequency depends on the configuration in respect of time of the delivered HF-power of the HF-generator (20) or on the configuration in respect of time of the HF-impedance between the electrodes (3, 4).

5. Device according to one of claims 1 to 4,
**characterised in that** the signal source emits an identification signal, when the electric HF-impedance, which has the body tissue between the two electrodes (3, 4) during a treatment process, exceeds a predefined value.

6. Device according to one of claims 1 to 4, **characterised in that** the signal source (30) emits an identification signal, when the emitted HF-power of the HF-generator (20) falls below a predefined value during a treatment process.

7. Device according to one of the preceding claims,
**characterised in that** the audible signal emitted by the signal source (30) changes suddenly, when the impedance of the body tissue between the electrodes (3, 4) rises above a predetermined value during a treatment process, or when the power output of the HF-generator (20) falls below a predefined value.

8. Device according to one of the preceding claims,
**characterised in that** the audible signal emitted by the signal source (30) is clocked, when the impedance of the body tissue between the electrodes (3, 4) during a treatment process rises above a predetermined value or when the power output of the HF-generator (20) falls below a predetermined value.

9. Device according to one claims 1 to 7,
**characterised in that** during a treatment process the signal source (30) taps from the HF-generator parameters proportional to the supplied HF-current and to the supplied HF-voltage, the supplied HF-power of the generator (20) and/or the HF-impedance between the electrodes (3, 4) is calculated from these parameters and a signal is emitted, which depends on the calculated delivered HF-power of the generator (20) and/or the calculated electrical HF-impedance which the body tissue has between the two electrodes (3, 4).

10. Device according to claim 1 or 3,
**characterised in that** a signal for regulating one of the electrical output parameters of the HF-generator (20) is derived from the signal of the signal source (30) which depends on the supplied HF-power of the HF-generator (20) or the electrical HF-impedance between the electrodes (3, 4), and is returned to the HF-generator (20).

11. Device according to claim 10,
**characterised in that** the power supplied by the HF-generator (20) is regulated in such a way that with an increase in the impedance value it is prevented from exceeding a predetermined value.

12. Device according to one of the preceding claims,
**characterised in that** the acoustic signal source (30) supplies a switch-off signal to the HF-generator (20), which separates or switches off the HF-generator (20) from the electrodes (3, 4), when the electrical impedance of the body tissue between the electrodes (3, 4) exceeds a predetermined absolute or relative value during a treatment process.

13. Device according to one of the preceding claims
**characterised in that** the signal source (30) supplies to the HF-generator (20) a signal, which reactivates the HF-generator (20) or connects it to the electrodes (3, 4), when the impedance of the body tissue between the electrodes (3, 4), after a preceding rise in impedance, falls below a predetermined absolute or relative value again.

14. Device according to one of the preceding claims,
**characterised in that** the supplied power from the HF-generator (20) is controlled in accordance with a time/power profile which can be predetermined by the user.

15. Device according to one of the preceding claims,
**characterised in that** the acoustic signal source (30) is contained in the HF-generator (20).

16. Device according to one of the preceding claims,
**characterised in that** the elongate applicator (2) which carries the electrodes (3, 4) is designed in the form of a bar with a constant or variable cross-section and runs to a point at its free front end.

## Revendications

1. Dispositif de traitement électrothermique du corps humain ou d'un corps animal, notamment pour la coagulation de tissus ou électrotomie,
ledit dispositif comportant un applicateur (1) de forme allongée comprenant
au moins deux électrodes (3, 4) destinées être introduites dans le corps à traiter, les deux électrodes (3, 4) étant disposées sur l'applicateur, à distance l'une de l'autre et en étant électriquement isolées l'une de l'autre, en vue de générer un champ électrique ou électromagnétique chauffant le tissu corporel dans la région de traitement et étant chacune reliées par une ligne à un générateur à hautes fréquences (20) placé à l'extérieur du corps,
et une source de signal (30) qui délivre un signal qui transmet à l'utilisateur pendant le processus de traitement une information sur l'état du tissu corporel situé entre les deux électrodes (3, 4),
**caractérisé en ce que** la fréquence du signal délivré par la source de signal (3, 4) augmente lorsque l'impédance du tissu corporel, situé entre les électrodes (3, 4), augmente pendant un processus de traitement.

2. Dispositif selon la revendication 1, **caractérisé en ce que** la fréquence du signal délivré par la source de signal (30) est à peu près proportionnelle à l'impédance du tissu corporel situé entre les électrodes (3, 4) ou inversement proportionnelle à la puissance HF du générateur HF (20).

3. Dispositif de traitement électrothermique du corps humain ou d'un corps animal, notamment pour la coagulation de tissus ou l'électrotomie,
ledit dispositif comportant un applicateur (1) de forme allongée comprenant
au moins deux électrodes (3, 4) destinées être introduites dans le corps à traiter, les deux électrodes (3, 4) étant disposées sur l'applicateur, à distance l'une de l'autre et en étant électriquement isolées l'une de l'autre, en vue de générer un champ électrique ou électromagnétique chauffant le tissu corporel dans la région de traitement et étant chacune reliées par une ligne à un générateur à hautes fréquences (20) placé à l'extérieur du corps,
et une source de signal (20) qui délivre un signal qui transmet à l'utilisateur pendant un processus de traitement une information sur l'état du tissu corporel situé entre les deux électrodes (3, 4),
**caractérisé en ce que** la fréquence du signal délivré par la source de signal (30) diminue lorsque l'impédance du tissu corporel entre les deux électrodes (3, 4) augmente.

4. Dispositif selon l'une des revendications précédentes, **caractérisé en ce que** la source de signal (30) est une source de signal acoustique qui délivre un signal audible dont la fréquence dépend de l'allure dans le temps de la puissance HF délivrée du générateur HF (20) ou de l'allure dans le temps de l'impédance entre les électrodes (3, 4).

5. Dispositif selon l'une des revendications 1 à 4, **caractérisé en ce que** la source de signal délivre un signal caractéristique lorsque l'impédance HF électrique, que présente le tissu corporel entre les deux électrodes (3, 4) pendant un processus de traitement, dépasse une valeur prescrite.

6. Dispositif selon l'une des revendications 1 à 4, **caractérisé en ce que** la source de signal (30) délivre un signal caractéristique lorsque la puissance HF délivrée du générateur HF (20) dépasse une valeur prescrite pendant le processus de traitement.

7. Dispositif selon l'une des revendications précédentes, **caractérisé en ce que** le signal audible délivré par la source de signal (30) varie brusquement si l'impédance du tissu corporel situé entre les électrodes (3, 4) dépasse une valeur prescrite pendant le processus de traitement ou si la puissance délivrée du générateur HF (20) tombe au-dessous d'une valeur prescrite.

8. Dispositif selon l'une des revendications précédentes, **caractérisé en ce que** le signal audible, qui est délivré par la source de signal (30), est cadencé lorsque l'impédance du tissu corporel situé entre les électrodes (3, 4) dépasse une valeur prescrite pendant un processus de traitement ou si la puissance délivrée du générateur HF (20) tombe au-dessous d'une valeur prescrite.

9. Dispositif selon l'une des revendications 1 à 7, **caractérisé en ce que** la source de signal (30) prend sur le générateur HF, pendant un processus de traitement, des grandeurs proportionnelles au courant délivré et à la tension HF délivrée, calcule à partir de ces grandeurs la puissance HF délivrée du générateur (20) et/ou l'impédance HF entre les électrodes (3, 4) et délivre un signal qui dépend de la puissance HF délivrée calculée du générateur (20) et/ou de l'impédance HF électrique calculée que présente le tissu corporel situé entre les deux électrodes (3, 4).

10. Dispositif selon la revendication 1 ou 3, **caractérisé en ce qu'**un signal de régulation de l'une des grandeurs de sortie électriques du générateur HF (20) est dérivé du signal de la source de signal (30), qui dépend de la puissance HF délivrée du générateur HF (20) ou de l'impédance HF électrique entre les électrodes (3, 4), et est ramené au générateur HF (20).

11. Dispositif selon la revendication 10, **caractérisé en ce que** la puissance délivrée du générateur HF (20) est régulée de telle sorte que, lorsque la valeur d'impédance augmente, il n'y pas de dépassement d'une valeur prescrite.

12. Dispositif selon l'une des revendications précédentes, **caractérisé en ce que** la source de signal acoustique (30) amène un signal de déconnexion au générateur HF (20) qui sépare ou déconnecte le générateur (20) des électrodes (3, 4) lorsque l'impédance électrique du tissu corporel situé entre les électrodes (3, 4) dépasse une valeur absolue ou relative prescrite pendant le processus de traitement.

13. Dispositif selon l'une des revendications précédentes, **caractérisé en ce que** la source de signal (30) amène au générateur HF (20) un signal qui active de nouveau le générateur HF (20) ou le relie aux électrodes (3, 4) si l'impédance du tissu corporel situé entre les électrodes (3, 4) tombe au-dessous d'une valeur absolue ou relative prescrite après que l'impédance a augmenté précédemment.

14. Dispositif selon l'une des revendications précédentes, **caractérisé en ce que** la puissance délivrée du générateur HF (20) est commandée conformément à un profil puissance/temps prescrit par l'utilisateur.

15. Dispositif selon l'une des revendications précédentes, **caractérisé en ce que** la source de signal acoustique (30) est contenue dans le générateur HF (20).

16. Dispositif selon l'une des revendications précédentes, **caractérisé en ce que** l'applicateur (2) de forme allongée, qui supporte les électrodes (3, 4), est conformé en barre de section constante ou variable et s'étend en pointe à son extrémité avant libre.
